# EUROPEAN PATENT APPLICATION

(11) **EP 4 494 732 A1**
(43) Date of publication of application: **22.01.2025**
(21) Application number: 22942871.9
(22) Date of filing: 05.07.2022
(51) Int. Cl.: B01D 39/16, A62B 23/00

(54) **METHOD FOR PRODUCING AN ANTIMICROBIAL NON-WOVEN FILTERING MATERIAL METALLIZED WITH SILVER**

(30) Priority: 14.05.2022 MD 20220027
(71) Applicant: Goncharov, Vitalii, Novosibirsk (RU); Poneatovschi, Ilia, Chisinau, MD-2001 (MD)
(72) Inventor: Goncharov, Vitalii, Novosibirsk (RU); Poneatovschi, Ilia, Chisinau, MD-2001 (MD)
(74) Representative: Vasilescu, Raluca
(86) International application number: PCT/MD2022/000001
(87) International publication number: WO 2023/224459

(57) **Abstract**

The invention relates to the metallization of a non-woven material. The antimicrobial non-woven filtering material is produced by converting metallic silver into its poorly soluble compounds, while a non-woven polypropylene-based web with a surface density of 15-90 g/m² continuously moves unwinding from a roll at a speed of 3...150 linear metres/min, and by continuously and successively wetting the web with different solutions using narrow-slotted fluid application nozzles having a working angle of 16...38°. Next, provisional pressing of surplus solutions and final combined drying of the finished material are performed. The technical result consists in the creation of a neutralizing filter for use in respiratory protection equipment, air conditioning and air handling systems, as well as medical garments, disposable bed sheets based on non-woven materials.

## Description

The invention relates to filtering materials having a long-acting antiviral and antibacterial activity designed for respiratory protection equipment, ventilation and air handling systems for food, educational premises, offices, production and other aseptic areas.

Silver has been one of the most studied materials over the past 100 years, also as the part of nanotechnologies. Being in the form of nanoparticles, including reduced one from aqueous solutions, silver in the metal form has much stronger and more stable antimicrobial, antiviral properties compared with its ions. Currently, silver is considered as one of the most important elements from a medical point of view. According to modern research, silver is a chemical element essential for good functioning of internal systems and organs as well as for enhancing immunity that actively affects pathogenic bacteria and viruses. The biocidal properties of silver and its compounds have been known for a long time. Silver is safe for human cells in small amounts but kills the vast majority of pathogenic bacteria and viruses transmitted through the air, so it has become widely used in controlling infections and treating humans. The effective antiviral and antibacterial properties of silver are relevant at the present time due to the emerging of large number of new dangerous strains of viruses and bacteria as well as the rate of mutations that are resistant to antibiotics and thus pose a serious threat to human life and health.

Filtering materials and articles thereof, in which silver in the form of ions is used as a bactericidal agent along with natural and synthetic antiseptics, are widely known.

A composite filtering material having an antimicrobial activity is known, wherein it contains a three-layer structure of silver nanoparticles on the surface of a carrier selected from the following: aluminosilicate, silicon dioxide, aluminium oxide, titanium dioxide [1], and is produced by the method comprising: cleaning the surface of the carrier selected from the following: aluminosilicate, silicon dioxide, aluminium oxide, titanium dioxide, treating the carrier with an aqueous solution of divalent tin salt, treating with an aqueous solution of silver salt and demineralized water, reduction of silver ions in the carrier phase until a layer containing silver nanoparticles is formed on its surface by photoactivation, washing and drying.

A disadvantage is that this material is effective for use in stationary and semi-stationary articles and is poorly suited for lightweight portable articles with a high degree of flexibility and mobility of the filtering layer, such as face bandages or dressing material, since the layer of the filtering material is placed in a frame due to its significant weight and the need to hold it in an upright position.

An antiviral filtering material [2] that is the closest to the invention is also known and it contains, as a volume-porous carrier, a layer of padding polyester metallized with silver having an initial surface density of 100-200 g/m2 and a volume density of 20-30 kg/m3, a thickness of at least 3 mm and a specific weight of silver of more than 0.1 g/g.

A disadvantage is that this antiviral material is effective in suppressing influenza A in a static position and has a little bactericidal effect on microbes and bacteria. Thus, it cannot be used in industrial production due to limited production volume resulted from using a galvanic manufacturing method (immersion), long time intervals for preparing the coating and high permeability of microorganisms through the finished product.

A general disadvantage is that these materials for filters are capable of capturing and retaining a large amount of pathogenic microflora on their surface without neutralizing them.

The aim of the present invention is to create an effective filtering material based on non-woven meltblown, spunbond web and their analogues with a specific weight of 15 to 80 g/m² having a high antimicrobial and antiviral activity by providing a flow-through, high-speed method of non-aerosol deposition of poorly soluble silver particles on a module for metallization operating in a flow-through mode with the material having its width of 50 to 1600 mm, and the web having its length of up to 2000 metres.

The method of modifying the material consists of depositing metallic silver particles onto the non-woven web, which is widely used in modern filtration systems and personal respiratory protection equipment, followed by converting silver into silver chloride or silver oxide.

The use of meltblown in personal protection equipment, such as "Medical face masks" and individual disposable respirators (N95, KN95 and their analogues), proved to be effective during seasonal outbreaks of respiratory diseases, including influenza group A. But such protection equipment also has a significant drawback, such as the possibility of use only by a healthy, non-infected person and the limited recommended time for use as protection equipment ranging from 30 minutes to 2 hours, after which pathogenic microflora accumulates on the inner surface and its growth occurs. The use of silver-containing materials in traumatology and orthopedics and in the treatment of infectious diseases allows dressing and filtering materials to have a significant long-acting disinfecting effect.

The module for metallization of the material surface with silver nanoparticles used in solving the problem has been developed and used for the first time, since existing metallization methods, such as vacuum deposition (small volume, increased energy costs), a galvanic method (small volume, long time process, length limitation of the material treated at one time), are not relevant in modern industry, which requires a flow-through and high-speed method for obtaining new non-woven materials.

The module for metallization allows applying nanodispersed compositions in two ways: volumetric and superficial. In the case of superficial treatment of the non-woven material, we achieve the most effective use of silver nanoparticles. But one should take into account the need for fine-tuning the concentration of solutions, including precursor solutions and reducing agent solutions, which directly depend on the density of the material, its structure and the desired rate at which the material is treated. One should also pay special attention to removing excess moisture while treating the material. Depending on the aim, the type and kind of the treated material, the module for metallization can be integrated into the existing technological chain of the production process. As a drive can be used non-woven web rewinding modules, bobbin-cutting, measuring and inspection machines, etc. A rewinding machine with a longitudinal material cutting function is used to operate the module. The machine is equipped with a material unwinding unit having a winding diameter of up to 0.6 metres and a dry material weight of up to 150 kilograms. It is equipped with a pneumatic tension control system for the material to be unwound, which directly affects the rate and quality of the coating. The winding unit is prepared for working with a non-woven material having a density of 15 to 90 g/m and with natural fabrics having a density of up to 440 g/m. It is equipped with sensors and provides a regulated constant material rewinding speed of 3 to 150 linear metres per minute necessary for the chemical reaction of surface metallization and for uniformity of the coating with a given density. Speed is important for optimizing the process of treatment and depends on the surface of the material, its hydrophobic properties and the concentration of the solutions used. The morphology and thickness of the resulting layer of silver nanoparticles on the surface also depend on the rate at which the material is treated with silver oxide or silver chloride.

The solutions are supplied to the treated material in a continuous mode through narrow-slotted, non-aerosol spray nozzles with a working angle of 16 to 38 degrees by means of circulating membrane pumps at an adjustable reagent supply rate of 9 to 55 litres per minute, while a minimum level of the residual reagent in the system for working with materials is maintained up to 1.6 metres wide. After each stage of treatment, the material passes through a direct squeezing system in order to remove residual solutions from the surface of the material and increase the service life of the reagent solutions.

The method for reduction of silver nanoparticles on the surface of the non-woven filtering material is performed in one pass through the module for metallization, and it comprises four stages of wet treatment:
a) preliminary preparation of the material;
b) metallization of the surface with a precursor solution (sensibilization);
c) chemical metallization with silver nitrate;
d) treatment with a reducing agent solution followed by removal of excess moisture and residual silver particles.

At the first stage (a), preliminary treatment of the non-woven material such as meltblown or spunbond is performed using an alkalescent solution with the addition of surfactants diluted in distilled water in order to degrease the surface of the material and convert the surface layer from a hydrophobic to a hydrophilic state necessary for subsequent reactions. Adding 20 % caustic soda and methyl or ethyl alcohol (up to 50 %) into the used solution affects the overall rate of the metallization process and the quality of preparation of the material surface, including through the pressure created by nozzles. The quality of the primary treatment significantly influences the degree of subsequent chemical reactions and the density of the material coating with silver nanoparticles.

At the second stage (b), preliminary metallization is performed using a precursor composition which consists of a copper nitrate trihydrate solution at the rate of 60-100 g/l of distilled water or a tin dihydrate solution at the rate of 10-35 g/l of distilled water with the addition of 10-50 ml of hydrochloric acid. After that, the material is squeezed to obtain residual surface moisture of 12-40 % and is heated to a temperature of 20-25 degrees.

At the third stage (c), silver is reduced from its nitrate salt using formalin, glucose, and ascorbic acid. The concentration of the silver nitrate solution is of great importance. If its concentration is below 1.5 g/l, the resulting coating is not formed, or it is of unsatisfactory quality. The best results are obtained with the silver nitrate concentration of 2.5 to 15 g/l, and at higher concentrations, silver precipitates on the surface in the form of white or metallic inclusions, making it difficult to obtain a distinct antibacterial coating. A method for silver reduction from an ammonia solution (25 %) at the rate of 25-50 ml/l of heated distilled water with the addition of caustic soda or caustic potash under constant stirring of the prepared concentrate.

At the fourth stage (d), treatment is performed using a reducing agent solution which consists of glucose of 10 to 50 g/l with the addition of formaldehyde at the rate of 5 to 50 ml/l of distilled water. Also, pyrogallol in combination with formaldehyde can be used as a reducing agent solution by pouring it through narrow-slotted nozzles in a non-aerosol manner under continuously supplying the solution in a closed cycle. After treating the material with the reducing agent solution, it is necessary to squeeze the material with ultrasonic rubberized rollers, which remove weakly fixed silver particles on the surface of the material and remove excess moisture before final drying of the material with infrared lamps.

The technical result consists in obtaining the material for filtration systems and upgrading existing ones to obtain new properties capable of neutralizing in a short time on its surface a large number of viruses and bacteria transmitted through the air, which pose a danger to human life and health. The filtering material treated with the silver compounds makes the surface of such material sterile. It improves the performance properties of the material increasing its service life and filtration quality. When used in medical face masks and disposable respirators, it gives the final product new, unique properties, also due to an increased period of continuous use, and can be a prototype for an individual human vaccination system.

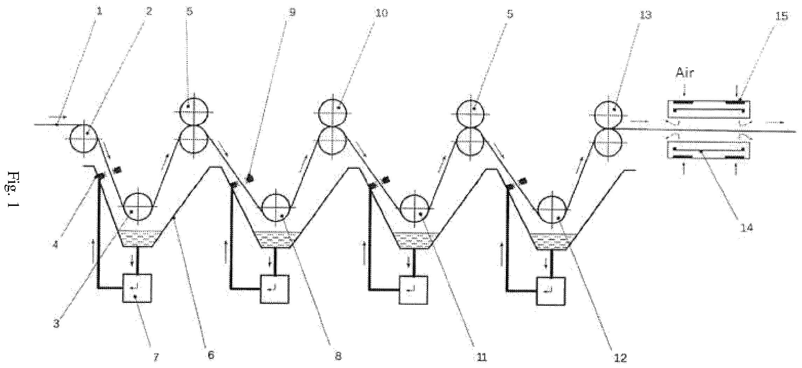

The invention is illustrated by drawings in Figs. 1-3, where:
Fig. 1 shows the scheme of implementation of the method for producing an antimicrobial non-woven filtering material metallized with silver;

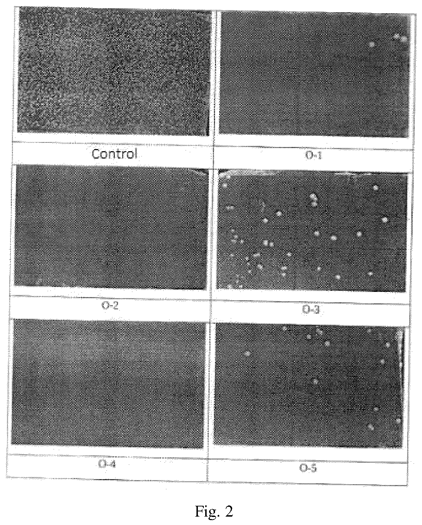

Fig. 2 shows antimicrobial activity of the elementary specimens from samples Nos. 1-2 against cells of the *Staphylococcus aureus* strain. Designations: "Control" designates formation of the colonies of the *Staphylococcus aureus* strain on agar plates in absence of contact with specimens from sample No. 1 (the average number was 1.5×10⁴ CFU/ml); O-1, O-2, O-3, O-4 and O-5 designate single colonies or their absence after contact of the *Staphylococcus aureus* strain culture seeded on an agar plate with specimens from samples Nos. 1-2 for 30 minutes;

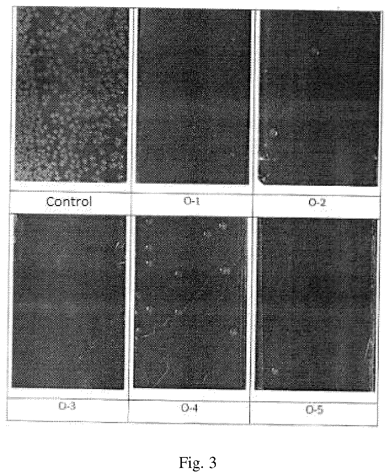

Fig. 3 shows antimicrobial activity of the elementary specimens from samples Nos. 1-2 against cells of the *E. coli* strain. Designations: "Control" designates formation of the colonies of the *E. coli* strain on agar plates in absence of contact with specimens from sample No. 1 (the average number was 1.0×10⁴ CFU/ml); O-1, O-2, O-3, O-4 and O-5 designate single colonies or their absence after contact of the *E. coli* strain culture seeded on an agar plate with specimens from samples Nos. 1-2 for 30 minutes.

The method is performed as follows (Fig. 1):
Meltblown non-woven material from a roll at an unwinding unit (not shown in the diagram) is threaded into a module and, bending around a row of rollers, is supplied to a web winding unit (not shown in the diagram).

### Stage a). Degreasing and dedusting of the web

A web 1 goes around the top of a guide roller 2 and, on the way to an impregnating roller 3, is irrigated from 2 sides through nozzles 4 with a solution of the following composition:
- sodium hydrocarbonate (NaHCO₃) at 10 g/l;
- caustic soda (NaOH) at 25 g/l;
- liquid soap (C₁₇H₃₅COOK) or OP-10 surfactant (C₉H₁₉C₆H₄O(C₂H₄O)₁₀OH) at 5 g/l;
- possibly 50 % ethyl alcohol (C₂H₅OH) at 500 g/l;
   then the web 1 goes around the bottom of the impregnating roller 3 and moves towards a pair of squeeze rollers 5. The roller 3 evenly distributes the impregnated liquid inside the web, and the rollers 5 remove surplus solution. Further, at all stages of the method, the moving web moves over solution collection and supply baths 6, from which closed-type circulating pumps 7 continuously supply the solutions to the nozzles 4 according to the stages and into which the excess volume of the solutions flows. In this way, the solutions in the baths 6 are continuously mixed, thereby maintaining constant concentrations of the solutions.

### Stage b). Metallization of the surface with a precursor solution (sensibilization)

The squeezed wet web 1 on the way to an impregnating roller 8 is irrigated from 2 sides through nozzles 9 with a solution of the following composition:
- tin chloride (SnCl₂) at 10 ... 35 g/l;
- hydrochloric acid (HCl) at 10 ... 50 g/l
   or
- copper nitrate trihydrate (CuH₆N₂O₉) at 60 ... 100 g/l;
then the web 1 goes around the bottom of the impregnating roller 8 and moves towards a pair of squeeze rollers 10 with heating.

### Stage c). Chemical metallization with silver nitrate

The squeezed wet and heated web 1 on the way to an impregnating roller 11 is irrigated from 2 sides through the nozzles 4 with a solution of the following composition:
- silver nitrate (AgNO₃) at 2.5 ... 15 g/l;
- 25 % aqueous ammonia (NH₄OH) at 25 ... 50 g/l;
- caustic soda (NaOH) or caustic potash (KOH) at 10 ... 30g/l;
then the web 1 goes around the bottom of the impregnating roller 11 and moves towards a pair of the squeeze rollers 5.

### Stage d). Reduction

The squeezed wet web 1 on the way to an impregnating roller 12 is irrigated from 2 sides through the nozzles 4 with a solution of the following composition:
- glucose (C₆H₁₂O₆) at 10 ... 50 g/l;
- formaldehyde (CH₂O) at 5 ...50 g/l;
then the web 1 goes around the bottom of the impregnating roller 12 and moves towards a pair of squeeze rollers 13 with ultrasonic drying and, on the way to a winding unit (not shown in the diagram), undergoes final drying with infrared lamps 14 in the air flow created by fans 15.

The coating obtained by the described method has high uniformity and strong antiviral properties, which expand the scope of application of the material used.

It should be noted that all reagents used for silver metallization must be of chemically pure grade. Replacing with analytical grade purity reagents will increase the consumption of the reagents during industrial production and reduce the activity time of the reagents.

The effectiveness of the obtained samples and the effect of the antibacterial coating were assessed by the Federal Budgetary Research Institution - State Research Centre of Virology and Biotechnology "VECTOR" of Rospotrebnadzor (Russia, Novosibirsk). Three material samples with different degrees of the coating were provided for testing.

"Determination of the virucidal and bactericidal activity of the materials for personal respiratory protection equipment (medical face masks) treated with silver compounds".
1. The virucidal activity of the materials was determined using the method of infection centres/focus forming units (FFU) adapted for this study (Hartshornet al., 2007).

### Materials and methods

### Objects of research

sample No. 1 - silver chloride, 50 %;
sample No. 2 - silver chloride, 100 %;
sample No. 3 - silver oxide, 100 %.

### Virus

Influenza virus A/Aichi/H3N2 was obtained from the State Collection of Microorganisms of the Federal Budgetary Research Institution - State Research Centre of Virology and Biotechnology "VECTOR" of Rospotrebnadzor.

### Determination of the infectious activity of the virus

To analyse the infectious activity of the influenza virus, the Madin-Darby Canine Kidney Cells (MDCK) continuous cell line was used. DMEM (Dulbecco Modification of Eagle Medium) with glucose and L-glutamine and 10 % inactivated fetal bovine serum (Hyclone) was used as a growth medium.

To obtain a confluent cell monolayer, 100 µl of cells at a concentration of 5×10⁵ cells/ml were added to each well of a 96-well plate and incubated for 24 h at a temperature of 37 °C, 95 % relative air humidity and 5 % CO₂ content.

To determine the infectivity of the virus, we used the method of infection centres/focus forming units (FFU) adapted for our study (Hartshornet al., 2007). Wells containing a MDCK cell monolayer were infected with 10-fold dilutions of the virus in the maintenance medium (DMEM with 2% fetal serum) at 100 µl. After incubation for 18 hours in a CO₂ thermostat at a temperature of 37 °C and 95 % relative air humidity, the medium was removed from the wells and the cell monolayer was fixed by adding per well 200 µl of diluted acetone (80 % acetone and 20 % distilled water) cooled to - 20 °C and incubating for 30 min. The cell monolayer was then washed with a phosphate-buffered saline (PBS) solution at pH 7.4 by adding 100 µl to each well. The cells were then stained with 1 % murine monoclonal antibodies to influenza virus nucleoprotein (CDC, Atlanta, GA, USA) at 50 µl per well and incubated at a temperature of 37 °C for 60 minutes. After incubation, the cells were washed three times with PBS and stained with 1 % anti-mouse IgG antibodies conjugated with horseradish peroxidase (Jackson Immuno Research Laboratories, Inc., West Grove, PA, USA) at 50 µl per well with incubation for 60 minutes at a temperature of 37 °C. The cells were then washed three times with PBS and the wells were treated with an AEC substrate solution (3-amino-9-ethylcarbazole, Sigma). The substrate was prepared as follows: 9 ml of distilled water, 200 µl of 0.5 M NaAc, 1 ml of AEC solution (2 AEC tablets were dissolved in 5 ml of DMSF), 50 µl of 3 % hydrogen peroxide. After 30 min of incubation in a dark room at room temperature, the substrate solution was removed and the plate was washed with PBS.

Influenza virus-infected cells stained red-brown were counted using a CKX41 Olympus inverted microscope at 200x magnification.

The virus titre was calculated using the Kerber method.

### Determination of the total amount of the influenza virus by quantitative PCR

Isolation of influenza virus RNA was performed using the QIAampViralRNAMiniKit (Qiagen, Germany) kit according to the manufacturer's protocol. Quantitative PCR was performed on a RotorGene 6000 (Corbett Research) using the following primers and probe (van Eldenetal., 2001): two forward primers 5'-GGA CTG CAG CGT AGA CGC TT-3' and 5'-CAT CCT GTT GTA TAT GAG GCC CAT-3'; a reverse primer 5'-CAT CT GTT GTA TAT GAG GCC CAT-3' and a probe 5'-FAM-CTC AGT TAT TCT GCT GGT GCA CTT GCC A-3' TAMRA.

Aliquots with the known RNA concentration were used in each PCR run as a positive control. A standard quantitative PCR curve using six 10-fold dilutions of the virus was used to estimate the variability in the total amount of the virus retained on the filtering material in each repetition.

### Aerosol experiment

4 ml of influenza virus A/Aichi 2/68 (A/H3N2) with an initial infectious titre of 7.50 Ig FFU/ml was poured into an "Omron" pneumatic sprayer that generated an aerosol with a median particle size of 1.1 µm and a polydispersity of 1.2. The sprayer was connected to a small dynamic chamber (SDC) with its volumetric air flow rate of 10 l/min, which was created by a sampler of MC-2 type. The fabric samples were mounted in a filter holder and placed inside the chamber, perpendicular to the aerosol flow. The viral suspension was sprayed for 10 min. The fabric sample was then removed from the SDC and cut into 1 cm × 1 cm pieces. After corresponding exposure for 0 min, 5 min, 15 min, 30 min and 60 min, following treatment with the viral aerosol, the fabric samples were placed in 1.7 ml Eppendorf tubes containing 1 ml of DMEM medium each and vortexed for 15 sec. After that, 140 µl of the medium was used for molecular genetic analysis by a real-time PCR method to determine the total amount of the virus deposited on the fabric, and the remaining part of the medium was used for virological testing to determine the amount of the viable (infectious) virus by the FFU method.

As a comparison sample was used fabric from ShB-1 "Lepestok-200" respirator approved for use when working with PBAs.

The results of the experiments are given in the table.

| No. | Specimen name | Exposure time, min | Concentration of the virus in the specimen, Ig FFU/ml |
|---|---|---|---|
| 1 | Silver chloride, 50 % "SCh" | 0 | 1.50 |
| 2 | | 5 | 1.47 |
| 3 | | 15 | 1.30 |
| 4 | | 30 | ≤ 1 |
| 5 | | 60 | ≤ 1 |
| 6 | Silver chloride, 100 % "SCh" | 0 | 1.54 |
| 7 | | 5 | 1.54 |
| 8 | | 15 | 1.37 |
| 9 | | 30 | ≤ 1 |
| 10 | | 60 | ≤ 1 |
| 11 | Silver oxide, 100 % "SO" | 0 | 1.20 |
| 12 | | 5 | ≤ 1 |
| 13 | | 15 | ≤ 1 |
| 14 | | 30 | ≤ 1 |
| 15 | | 60 | ≤ 1 |
| 16 | -1 "-200" (ShB-1 "Lepestok - 200") respirator | 0 | 5.54 |
| 17 | | 5 | 4.84 |
| 18 | | 15 | 4.47 |
| 19 | | 30 | 3.69 |
| 20 | | 60 | 3.36 |
| | Virus control using MC-2 | | 5.43 |
| Initial | A/Aichi 2/68 (A/H3N2) | | 7.64 |

The table shows the average values for four repetitions.

Conclusions in respect of the results of the virucidal activity study of samples Nos. 1-3 based on 50 % silver chloride, 100 % silver chloride, 100 % silver oxide:
- the fabric treated with the silver compounds of all studied samples has a strong virucidal activity; at the time point "0", the concentration of the infective virus on the fabric in all studies is by 4 orders of magnitude lower than when the same total amount of the virus enters sorbent liquid of the MC-2 sampler;
- no infective virus is detected after 30 min following retention of the viral aerosol on the fabric surface treated with silver chloride at a concentration of 50-100 %;
- the fabric treated with silver chloride at a concentration of 100 % showed a virucidal activity comparable to that of the fabric treated with 50 % silver chloride;
- on the fabric treated with 100 % silver oxide at the initial time point 0, the infective virus is detected at a concentration lower than on the fabrics treated with silver chloride, and at the following time control points, no live virus is detected;
- the fabric which serves as a material for the ShB-1 "Lepestok-200" respirator did not show any virucidal activity. The concentration of the infective virus on the fabric is comparable to that in the sampler.

2. The bactericidal activity of the materials was determined by the method for determination of the microbial permeability of materials treated with silver compounds using agar media according to GOST 12.4.136-84 "Method for determination of the microbial permeability".

### Materials and methods

### Objects of research

sample No. 1 - fabric treated with 50 % silver chloride;
sample No. 2 - fabric treated with 100 % silver chloride;
sample No. 1 - fabric treated with 100 % silver oxide.

### Test strains of microorganisms

- *Staphylococcus aureus* B-1266
- *Escherichia coli* B-655

### Experimentation

### 1. Preparation of working culture suspensions of the test strains

3 ml of sterile saline solution was added to a tube containing a 24-hour test culture grown on nutrient agar and, through rotating the tube between the palms, the grown colonies of microorganisms were washed off. The resulting cell suspension was then adjusted to a concentration of 1 billion microbial bodies per 1 ml using saline solution in accordance with the turbidity standard for optical standardization of bacterial slurries. Next, 10-fold dilutions of the resulting suspension were prepared to obtain a test culture concentration of 1000 microbial bodies in 1 ml.

### 2. Preparation of agar plates

3.5 ml of nutrient medium was poured onto each of thirty glass plates, 25×40×2 mm in size, placed in Petri dishes, and evenly distributed over entire surface of the plate. All manipulations were performed under aseptic conditions.

### 3. Determination of the antimicrobial effect

3.1. Using a micropipette, 0.1 ml of the test culture suspension was applied onto eighteen glass plates with the nutrient medium, six of which were controls, and it was evenly distributed over the entire surface of the nutrient medium using a spatula and kept in a thermostat for 15 min at a temperature of 37 °C. The control plates were left in the thermostat for 24 h at a temperature of 37 °C.

3.2. Under aseptic conditions, elementary specimens from sample No. 1 were placed using tweezers onto the remaining twelve glass plates with the nutrient medium.

3.3. The glass plates with the nutrient medium and test culture were placed on top of the elementary specimens, the Petri dishes were closed and kept in the thermostat for 30 min.

3.4. After 30 min, the elementary specimens along with the plates with the nutrient medium and test culture were removed from the Petri dishes. The plates with the nutrient medium remaining in the Petri dishes were placed into the thermostat at a temperature of 37 °C for 24 h to grow microorganisms that had penetrated through the elementary specimens.

3.5. After 24 h, a colony counting device was used to determine the number of colonies of microorganisms that had grown on six control plates and twelve experimental plates with penetrated microorganisms. The results of the experiments are presented in Tables 1-7. The tables show the average values for four repetitions.

**Table 1. Determination of the antimicrobial effect of the elementary specimens (sample No. 1) on cells of the Staphylococcus aureus B-1266 test culture**

| Variant | Number of the specimens | Dilution | Volume of the seeded suspension | CFU in 100 µl | Average CFU in 1 ml |
|---|---|---|---|---|---|
| Control | 1 | 4 | 0.1 ml | 1500 | 1.6 × 10⁴ |
| | 2 | -«- | -«- | 1600 | |
| | 3 | -«- | -«- | 1700 | |
| | 4 | -«- | -«- | 1800 | |
| | 5 | -«- | -«- | 1500 | |
| | 6 | -«- | -«- | 1500 | |
| Experiment | 1 | 4 | 0.1 ml | 3 | 1.9 × 10 |
| | 2 | -«- | -«- | 0 | |
| | 3 | -«- | -«- | 0 | |
| | 4 | -«- | -«- | 2 | |
| | 5 | -«- | -«- | 3 | |
| | 6 | -«- | -«- | 4 | |
| | 7 | -«- | -«- | 0 | |
| | 8 | -«- | -«- | 0 | |
| | 9 | -«- | -«- | 0 | |
| | 10 | -«- | -«- | 7 | |
| | 11 | -«- | -«- | 0 | |
| | 12 | -«- | -«- | 4 | |

**Table 2. Determination of the antimicrobial effect of the elementary specimens (sample No. 1) on cells of the E. coli B-655 test culture**

| Variant | Number of the specimens | Dilution | Volume of the seeded suspension | CFU in 100 µl | Average CFU in 1 ml |
|---|---|---|---|---|---|
| Control | 1 | 4 | 0.1 ml | 328 | 3.8 × 10³ |
| | 2 | -«- | -«- | 440 | |
| | 3 | -«- | -«- | 350 | |
| | 4 | -«- | -«- | 387 | |
| | 5 | -«- | -«- | 416 | |
| | 6 | -«- | -«- | 376 | |
| Experiment | 1 | 4 | 0.1 ml | 2 | 3.25 × 10 |
| | 2 | -«- | -«- | 0 | |
| | 3 | -«- | -«- | 7 | |
| | 4 | -«- | -«- | 10 | |
| | 5 | -«- | -«- | 0 | |
| | 6 | -«- | -«- | 1 | |
| | 7 | -«- | -«- | 1 | |
| | 8 | -«- | -«- | 0 | |
| | 9 | -«- | -«- | 4 | |
| | 10 | -«- | -«- | 6 | |
| | 11 | -«- | -«- | 5 | |
| | 12 | -«- | -«- | 3 | |

**Table 3. Determination of the antimicrobial effect of the elementary specimens (sample No. 2) on cells of the Staphylococcus aureus B-1266 test culture**

| Variant | Repetition | Dilution | Volume of the seeded suspension | CFU in 100 µl | Average CFU in 1 ml |
|---|---|---|---|---|---|
| Control | 1 | 4 | 0.1 ml | 1300 | 1.3 × 10⁴ |
| | 2 | -«- | -«- | 1280 | |
| | 3 | -«- | -«- | 1103 | |
| | 4 | -«- | -«- | 1446 | |
| | 5 | -«- | -«- | 1254 | |
| | 6 | -«- | -«- | 1249 | |
| Experiment | 1 | 4 | 0.1 ml | 2 | 1.3 × 10 |
| | 2 | -«- | -«- | 4 | |
| | 3 | -«- | -«- | 2 | |
| | 4 | -«- | -«- | 3 | |
| | 5 | -«- | -«- | 0 | |
| | 6 | -«- | -«- | 2 | |
| | 7 | -«- | -«- | 0 | |
| | 8 | -«- | -«- | 0 | |
| | 9 | -«- | -«- | 1 | |
| | 10 | -«- | -«- | 2 | |
| | 11 | -«- | -«- | 0 | |
| | 12 | -«- | -«- | 0 | |

**Table 4. Determination of the antimicrobial effect of the elementary specimens (sample No. 2) on cells of the E. coli B-655 test culture**

| Variant | Repetition | Dilution | Volume of the seeded suspension | CFU in 100 µl | Average CFU in 1 ml |
|---|---|---|---|---|---|
| Control | 1 | 4 | 0.1 ml | 1284 | 1.3 × 10⁴ |
| | 2 | -«- | -«- | 1348 | |
| | 3 | -«- | -«- | 1190 | |
| | 4 | -«- | -«- | 1401 | |
| | 5 | -«- | -«- | 1322 | |
| | 6 | -«- | -«- | 1346 | |
| Experiment | 1 | 4 | 0.1 ml | 0 | 7.5 |
| | 2 | -«- | -«- | 1 | |
| | 3 | -«- | -«- | 0 | |
| | 4 | -«- | -«- | 0 | |
| | 5 | -«- | -«- | 0 | |
| | 6 | -«- | -«- | 2 | |
| | 7 | -«- | -«- | 1 | |
| | 8 | -«- | -«- | 1 | |
| | 9 | -«- | -«- | 0 | |
| | 10 | -«- | -«- | 2 | |
| | 11 | -«- | -«- | 0 | |
| | 12 | -«- | -«- | 2 | |

**Table 5. Determination of the antimicrobial effect of the elementary specimens (sample No. 3) on cells of the Staphylococcus aureus B-1266 test culture**

| Variant | Repetition | Dilution | Volume of the seeded suspension | CFU in 100 µl | Average CFU in 1 ml |
|---|---|---|---|---|---|
| Control | 1 | 4 | 0.1 ml | 625 | 6.3 × 10³ |
| | 2 | -«- | -«- | 740 | |
| | 3 | -«- | -«- | 578 | |
| | 4 | -«- | -«- | 604 | |
| | 5 | -«- | -«- | 532 | |
| | 6 | -«- | -«- | 701 | |
| Experiment | 1 | 4 | 0.1 ml | 0 | 0 |
| | 2 | -«- | -«- | 0 | |
| | 3 | -«- | -«- | 0 | |
| | 4 | -«- | -«- | 0 | |
| | 5 | -«- | -«- | 0 | |
| | 6 | -«- | -«- | 0 | |
| | 7 | -«- | -«- | 0 | |
| | 8 | -«- | -«- | 0 | |
| | 9 | -«- | -«- | 0 | |
| | 10 | -«- | -«- | 0 | |
| | 11 | -«- | -«- | 0 | |
| | 12 | -«- | -«- | 0 | |

**Table 6. Determination of the antimicrobial effect of the elementary specimens (sample No. 3) on cells of the E. coli B-655 test culture**

| Variant | Repetition | Dilution | Volume of the seeded suspension | CFU in 100 µl | Average CFU in 1 ml |
|---|---|---|---|---|---|
| Control | 1 | 4 | 0.1 ml | 1220 | 1.2 × 10⁴ |
| | 2 | -«- | -«- | 1301 | |
| | 3 | -«- | -«- | 1105 | |
| | 4 | -«- | -«- | 1256 | |
| | 5 | -«- | -«- | 1050 | |
| | 6 | -«- | -«- | 986 | |
| Experiment | 1 | 4 | 0.1 ml | 0 | 1.3 × 10 |
| | 2 | -«- | -«- | 0 | |
| | 3 | -«- | -«- | 0 | |
| | 4 | -«- | -«- | 0 | |
| | 5 | -«- | -«- | 0 | |
| | 6 | -«- | -«- | 0 | |
| | 7 | -«- | -«- | 0 | |
| | 8 | -«- | -«- | 3 | |
| | 9 | -«- | -«- | 7 | |
| | 10 | -«- | -«- | 0 | |
| | 11 | -«- | -«- | 6 | |
| | 12 | -«- | -«- | 0 | |

**Table 7. Determination of the antimicrobial effect of the elementary specimens (samples Nos. 1-3) on cells of the Staphylococcus aureus B-1266 and E. coli B-655 test cultures**

| Variant of material treatment | Strain / Number of cells in the control and experiment (CFU/ml) | | | |
|---|---|---|---|---|
| | *S. aureus* | | *E. coli.* | |
| | Control (non-treated) | Experiment | Control (non-treated) | Experiment |
| Silver chloride, 50 % | 1.6 × 10⁴ | 1.9 × 10 | 3.8 × 10³ | 2.6 × 10 |
| Silver chloride, 100 % | 1.3 × 10⁴ | 1.3 × 10 | 1.3 × 10⁴ | 7.5 |
| Silver oxide, 100 % | 6.3 × 10³ | 0 | 1.2 × 10⁴ | 1.3 × 10 |

| | | | | |
|---|---|---|---|---|
| Conclusions in respect of the results of the bactericidal activity study of samples Nos. 1-3 based on 50 % silver chloride, 100 % silver chloride, 100 % silver oxide | | | | |

Based on the experimental studies of three samples of the materials intended for the manufacture of medical face masks, treated with the silver compounds, it was found that treatment of the material with the applied silver compounds reduced the number of viable microorganisms in the experiments on average by three orders of magnitude, i. e. 1000 times (Fig. 2, 3), compared to the control cell suspension, while the antibacterial activity of the material treated with 100 % silver chloride turned out to be comparable to that of the fabric treated with 50 % silver chloride (Tables 1-7).

The material treated with 100 % silver oxide showed the highest antistaphylococcal activity among the silver compounds studied. While the concentration of *S*. *aureus* cells in the control amounted to 6.3 × 10³ cells/ml, the number of *S. aureus* cells in the experiment dropped to zero.

According to the data obtained, it can be concluded that all three studied samples of the material based on 50-100 % silver chloride and 100 % silver oxide have a high statistically significant antibacterial activity against *S*. *aureus* and *E. coli* cells.

## Claims

1. A method for producing an antimicrobial non-woven filtering material metallized with silver, providing conversion of metallic silver into its poorly soluble compounds, while a non-woven polypropylene-based web with a surface density of 15-90 g/m² continuously moves unwinding from a roll at a speed of 3... 150 linear metres/min, and by continuously and successively wetting the web with different solutions using narrow-slotted fluid application nozzles having a working angle of 16...38°, followed by provisional squeezing of surplus solutions and final combined drying of the finished material, comprising the following operations:
a) degreasing and dedusting of the web with a solution of the following composition:
• sodium hydrocarbonate (NaHCO₃) at 10 g/l;
• caustic soda (NaOH) at 20 ... 38 g/l;
• liquid soap (C₁₇H₃₅COOK) or OP-10 surfactant (C₉H₁₉C₆H₄O(C₂H₄O)₁₀OH) at 3 ... 5 g/l;
• possibly 50 % ethyl alcohol (C₂H₅OH) at 200 ... 500 g/l;
b) metallization of the surface with a precursor solution (sensibilization) of the following composition:
• tin chloride (SnCl₂) at 10 ... 35 g/l;
• hydrochloric acid (HCl) at 10 ... 50 g/l
• or
• copper nitrate trihydrate (C_{U}H₆N₂O₉) at 60 ... 100 g/l;
c) chemical metallization with a silver nitrate solution of the following composition:
• silver nitrate (AgNO₃) at 2.5 ... 15 g/l;
• 25 % aqueous ammonia (NH₄OH) at 25 ... 50 g/l;
• caustic soda (NaOH) or caustic potash (KOH) at 10 ... 30 g/l;
d) reduction with a solution of the following composition:
• glucose (C₆H₁₂O₆) at 10 ... 50 g/l;
• formaldehyde (CH₂O) at 5 ...50 g/l.

2. The method according to claim 1, wherein the provisional squeezing of the moving web during each operation is performed to obtain residual surface moisture of 12-40 %.

3. The method according to claim 1, wherein the moving web is heated to a temperature of 20-25 °C during operation c).

4. The method according to claim 1, wherein the final drying is performed by infrared radiation with constant airflow.

5. An antimicrobial filtering material containing the non-woven material having the surface of the fibres coated with a layer of a poorly soluble silver compound in the form of silver chloride or silver oxide obtained by the method described in claim 1.
